# EUROPEAN PATENT APPLICATION

(11) **EP 4 385 498 A1**
(43) Date of publication of application: **19.06.2024**
(21) Application number: 22866656.6
(22) Date of filing: 07.09.2022
(51) Int. Cl.: A61K 9/19, A61K 31/40, A61P 35/00, A61P 37/02, A61P 9/00

(54) **PHARMACEUTICAL PREPARATION, PREPARATION METHOD THEREFOR AND USE THEREOF**

(30) Priority: 08.09.2021 CN 202111051190
(71) Applicant: Coherent Biopharma (Suzhou), Limited, Industrial Park Suzhou Jiangsu 215123 (CN)
(72) Inventor: FAN, Sixiang, Suzhou, Jiangsu 215123 (CN); SHANG, Peipei, Suzhou, Jiangsu 215123 (CN); ZHANG, Xianhui, Suzhou, Jiangsu 215123 (CN)
(74) Representative: Maiwald GmbH
(86) International application number: PCT/CN2022/117610
(87) International publication number: WO 2023/036193

(57) **Abstract**

A pharmaceutical preparation, containing a ligand-drug conjugate, a lyophilized excipient and a buffer. The preparation is subjected to pH screening, the buffer and the lyophilized excipient used therein are defined, and the freeze-drying process is also screened, so that the appearance, the water content, the impurity content, the re-dissolution time, etc. of the preparation during storage can be kept stable.

## Description

### Priority and Related Application

The present application claims the right of priority for Chinese patent application no. 202111051190.6, filed with the China National Intellectual Property Administration on September 8, 2021 and entitled "PHARMACEUTICAL PREPARATION, PREPARATION METHOD THEREFOR AND USE THEREOF", which is incorporated herein by reference in its entirety.

### Technical Field

The present invention relates to the field of biomedicine. In particular, the present invention relates to a pharmaceutical preparation, in particular to a pharmaceutical preparation comprising a ligand-drug conjugate, and a preparation method therefor and the use thereof.

### Background Art

There are a variety of specific or overexpressed receptors, such as transferrin receptor (TFR), low density lipoprotein receptor, folate receptor, urokinase receptor, tumor necrosis factor receptor, intercellular adhesion molecule (ICAM) and integrin receptor LFA-1, on the surface of tumor cells and other diseased cells (such as vascular endothelial cells, leukocytes and brain capillary endothelial cells). Ligands of these receptors can be divided into proteins (ferritin, apolipoprotein, low density lipoprotein receptor-related protein 1 (LRP1), *etc.*), polypeptides (insulin, SOR-C13, luteinizing hormone releasing hormone (LHRH), somatostatin SST-14, *etc.*), and small molecules (folic acid and analogs, carbohydrates). These ligands have the characteristics of good specificity, moderate affinity, and obvious biological effect when binding to receptors and can obviously improve the targeting property and efficacy of the drugs while reducing the toxicity when coupling with therapeutic drugs to form ligand-drug conjugates (LDCs). The ligand-drug conjugate has endocytosis mediation function, can be used in the immune regulation and the treatment of tumors and cardiovascular diseases, and is advantageous in that: (1) targeting and endocytosis structures are combined, which can realize that any ligand-targeted polypeptide is used as a guidance moiety, thereby broadening the targeting range of such drugs; (2) the affinity and targeting property of the drug conjugate to diseased cells are enhanced utilizing a dual-targeting ligand, such that an efficient toxin drug such as monomethyl auristatin E (MMAE) can be carried, thereby enhancing the efficacy, broadening the therapeutic window to avoid the side effect of the drug; and (3) Linkers make the conjugate unable to release drug molecules outside cells (intercellular substances, blood circulation system, *etc.*), thereby ensuring the stability of the drug in the circulation *in vivo*, reducing the drug toxicity and not generating toxic effects on normal cells. After entering targeted cells, the linker is cleaved to release the drug molecules with a therapeutic effect, thereby avoiding the generation of multiple drug resistance (MDR).

The LDC preparation needs to be formulated in a way that keeps its stability during storage and subsequent use, in addition to requiring the conjugate to be formulated in a manner suitable for administration to a subject. For example, if the LDC is not properly formulated in a liquid, the LDC in the liquid solution tends to decompose, aggregate, or undergo undesirable chemical modifications, *etc.* The stability of the LDC in a preparation depends on the buffering agent, stabilizer, surfactant, *etc.* used in the preparation.

There is a need in the art for novel pharmaceutical preparations comprising LDC that is sufficiently stable and suitable for administration to a subject. Therefore, suitable LDC preparations need to be prepared for the treatment or prevention of diseases.

### Summary of the Invention

### Problems to be solved by the invention

With regard to the above-mentioned need in the art for novel pharmaceutical preparations comprising LDC that is stable and suitable for administration to a subject, the present invention is intended to provide a preparation comprising a ligand-drug conjugate, a preparation method therefor and the use thereof. By defining parameters such as pH value, buffering agent, lyophilized excipient and freeze-drying process, the need of keeping good stability during storage of a LDC preparation is met.

### Solutions for solving problems

In a first aspect, the present invention provides a pharmaceutical preparation, comprising an active drug and optionally a pharmaceutically acceptable adjuvant.

Further, in the above-mentioned pharmaceutical preparation, the active drug comprises a ligand-drug conjugate or a pharmaceutically acceptable salt thereof; the pharmaceutically acceptable adjuvant comprises one or more of a lyophilized excipient and a buffering agent.

Further, in the above-mentioned pharmaceutical preparation, the pharmaceutically acceptable adjuvant further comprises a solvent.

Further, in the above-mentioned pharmaceutical preparation, the ligand-drug conjugate is a dual ligand-drug conjugate; preferably, the dual ligand-drug conjugate is a drug conjugate targeting a folate receptor and a TRPV6 receptor; more preferably, the dual ligand-drug conjugate has the following structure: 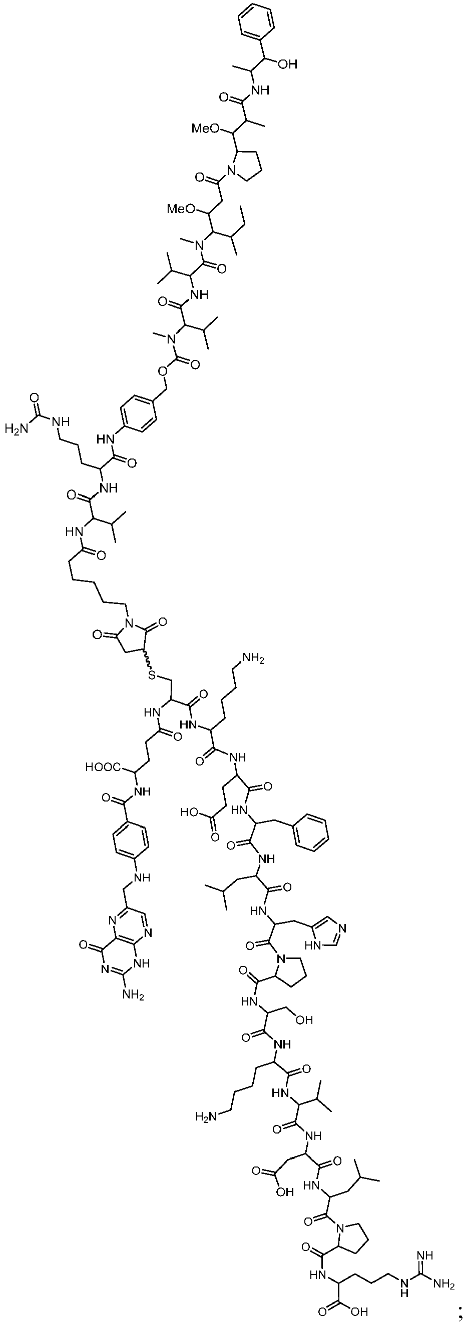 and further preferably, the dual ligand-drug conjugate has the following structure:

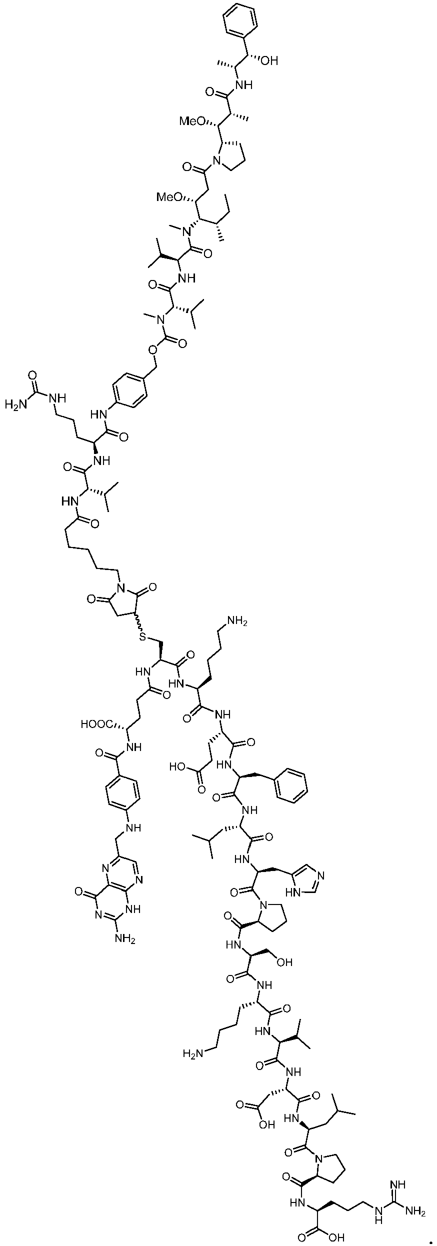

Further, in the above-mentioned pharmaceutical preparation, the solvent is water; preferably, the solvent is purified water; more preferably, the solvent is sterile water, distilled water or deionized water; and more preferably, the sterile water is sterile water for injection, single-distilled water, or double-distilled water.

Further, in the above-mentioned pharmaceutical preparation, the active drug has a concentration of 4.75 mg/mL to 6 mg/mL; and preferably, the active drug has a concentration of 4.75 mg/mL, 5 mg/mL, 5.25 mg/mL, 5.5 mg/mL, 5.75 mg/mL, or 6 mg/mL.

Further, the above-mentioned pharmaceutical preparation has a pH value of 6.5 to 8.5; preferably, the pharmaceutical preparation has a pH value of 6.9 to 8.0, such as 6.9, 7.0, 7.1, 7.2, 7.3, 7.4, 7.5, 7.6, 7.7, 7.8, 7.9, or 8.0; and more preferably, the pharmaceutical preparation has a pH value of 6.9 to 7.7, such as 6.9, 7.0, 7.3, 7.5, or 7.7.

Further, in the above-mentioned pharmaceutical preparation, the lyophilized excipient comprises a polyol, such as one or more of mannitol, sorbitol, inositol, and xylitol; and preferably, the lyophilized excipient comprises mannitol.

Further, in the above-mentioned pharmaceutical preparation, the lyophilized excipient further comprises saccharides, such as one or more of a monosaccharide, a disaccharide, and a polysaccharide; preferably, the mass ratio of the saccharides to the polyol is 1:1 to 1:5; and more preferably, the mass ratio of the saccharides to the polyol is 1:2 to 1:4.

Preferably, in the above-mentioned pharmaceutical preparation, the monosaccharide comprises one or more of glucose and fructose; the disaccharide comprises one or more of sucrose, trehalose, maltose, and lactose; and the polysaccharide comprises one or more of cyclodextrin and dextran.

More preferably, in the above-mentioned pharmaceutical preparation, the lyophilized excipient comprises mannitol and sucrose; preferably, the mass ratio of the sucrose to the mannitol is 1:2 to 1:5, such as 1:2, 1:2.4, 1:2.5, 1:4, 1:4.5, 1:4.7, or 1:5; and more preferably, the mass ratio of the sucrose to the mannitol is 1:4.

More preferably, in the above-mentioned pharmaceutical preparation, the lyophilized excipient comprises mannitol and trehalose; preferably, the mass ratio of the trehalose to the mannitol is 1:2 to 1:4; and more preferably, the mass ratio of the trehalose to the mannitol is 1:4.

Further, in the above-mentioned pharmaceutical preparation, the buffering agent comprises tromethamine and an acidic substance; and the mass ratio of the tromethamine to the active drug is 1:4 to 1:10, preferably 1:5 to 1:8.5.

Preferably, in the above-mentioned pharmaceutical preparation, the acidic substance comprises one or more of hydrochloric acid, acetic acid, sodium dihydrogen phosphate, and tromethamine hydrochloride.

More preferably, in the above-mentioned pharmaceutical preparation, the buffering agent comprises tromethamine and hydrochloric acid; preferably, the mass ratio of the tromethamine to the active drug is 1:5 to 1:8.5, such as 1:5, 1:5.5, 1:6, 1:6.5, 1:7, 1:8, or 1:8.5; and more preferably, the ratio of the tromethamine to the active drug is 1:6.5.

Further, in the above-mentioned pharmaceutical preparation, each 1 mL of the pharmaceutical preparation comprises 4.75-6 mg of a ligand-drug conjugate or a pharmaceutically acceptable salt thereof, 25-55 mg of mannitol, 0-55 mg of sucrose, 0.5-1.25 mg of tromethamine, hydrochloric acid, and the balance made up of a solvent; wherein the pharmaceutical preparation has a pH value of 6.5-8.5, preferably 6.9 to 8.0; for example, each 1 mL of the pharmaceutical preparation comprises 5 mg of a ligand-drug conjugate or a pharmaceutically acceptable salt thereof, 40 mg of mannitol, 10 mg of sucrose, 0.765 mg of tromethamine, hydrochloric acid, and the balance made up of water; wherein the pharmaceutical preparation has a pH value of 6.5-8.5, preferably 6.9 to 8.0.

Further, the above-mentioned pharmaceutical preparation is sterile.

Further, the above-mentioned pharmaceutical preparation is stable during freezing and thawing.

In a second aspect, the present invention provides a preparation method for preparing the above-mentioned pharmaceutical preparation, comprising the following steps: using a prescription amount of an active drug and optionally adding a pharmaceutically acceptable adjuvant, and carrying out uniform mixing to obtain the pharmaceutical preparation.

Further, the above-mentioned preparation method comprises the following steps: using a prescription amount of a ligand-drug conjugate or a pharmaceutically acceptable salt thereof, a lyophilized excipient, a buffering agent and a solvent, dissolving the ligand-drug conjugate or the pharmaceutically acceptable salt thereof and the lyophilized excipient using the solvent, adjusting the pH value to 6.9 to 7.7 using the buffering agent, carrying out uniform mixing, and carrying out filtering to obtain the pharmaceutical preparation.

In a third aspect, the present invention provides a freeze-dried preparation, wherein the freeze-dried preparation is prepared by freeze-drying the above-mentioned pharmaceutical preparation.

In a fourth aspect, the present invention provides a preparation method for preparing the above-mentioned freeze-dried preparation, comprising the following steps: (i) freezing the pharmaceutical preparation at -45°C; (ii) annealing the pharmaceutical preparation at -25°C; (iii) re-freezing the pharmaceutical preparation at -45°C; (iv) carrying out first drying on the pharmaceutical preparation at -15°C; and (v) carrying out second drying on the pharmaceutical preparation at 25°C.

Further, in the above-mentioned preparation method, the time of the first drying is 800 to 3000 min; and the time of the second drying is 300 to 600 min.

In a fifth aspect, the present invention provides a liquid preparation reconstituted by the above-mentioned freeze-dried preparation using water.

Further, in the above-mentioned liquid preparation, the water comprises one or more of distilled water, pure water, and sterile water; and preferably, the freeze-dried preparation has a pH value of 6.5 to 8.5 after being reconstituted using water.

In a sixth aspect, the present invention provides a preparation method for preparing the above-mentioned liquid preparation, comprising the following steps: mixing the freeze-dried preparation with sterile water to obtain the liquid preparation.

In a seventh aspect, the present invention provides a drug-containing delivery device comprising one of the following preparations: the above-mentioned pharmaceutical preparation, the above-mentioned freeze-dried preparation or the above-mentioned liquid preparation.

In an eighth aspect, the present invention provides a pre-filled syringe comprising one of the following preparations: the above-mentioned pharmaceutical preparation, the above-mentioned freeze-dried preparation or the above-mentioned liquid preparation, preferably for use in intravenous injection or intramuscular injection.

In a ninth aspect, the present invention provides the use of the above-mentioned pharmaceutical preparation, the above-mentioned freeze-dried preparation and the above-mentioned liquid preparation in the preparation of a delivery device or a pre-filled syringe or a drug for enhancing an immune effector cell response and/or reducing immunosuppression in a subject.

In a tenth aspect, the present invention provides the use of the above-mentioned pharmaceutical preparation, the above-mentioned freeze-dried preparation and the above-mentioned liquid preparation in the preparation of a delivery device or a pre-filled syringe or a drug for the treatment or prevention of a cancer, an immune disease, a cardiovascular disease, a metabolic disease and a neurological disease in a subject.

Further, in the above-mentioned use, the cancer comprises one or more of breast cancer, lung cancer, prostate cancer, kidney cancer, leukemia, ovarian cancer, stomach cancer, uterine cancer, endometrial cancer, liver cancer, colon cancer, thyroid cancer, pancreatic cancer, colorectal cancer, esophageal cancer, skin cancer, lymphoma, and multiple myeloma; the immune disease comprises one or more of a connective tissue disease, systemic sclerosis, rheumatoid arthritis, and systemic lupus erythematosus; the cardiovascular disease comprises one or more of angina pectoris, myocardial infarction, apoplexy, heart attack, a hypertensive heart disease, a rheumatic heart disease, cardiomyopathy, cardiac arrhythmia, and a congenital heart disease; the metabolic disease comprises one or more of diabetes mellitus, gout, obesity, hypoglycemia, hyperglycemia, and dyslipidemia; and the neurological disease comprises one or more of Alzheimer's disease, Parkinson's disease, Huntington's disease, head injury, multiple sclerosis, vertigo, coma, and epilepsy.

### Effects of the Invention

In the present invention, the preparation containing the ligand-drug conjugate is subjected to pH screening, the buffering agent and the lyophilized excipient used therein are defined, and the freeze-drying process is also screened, so that the appearance, the water content, the impurity content, the re-dissolution time, *etc.* of the preparation during storage can be kept stable.

### Detailed Description of Embodiments

Embodiments of the present invention are described below, but the present invention is not limited thereto. The present invention is not limited to each composition described below. Various modifications can be made within the scope of protection of the present invention. Embodiments and examples obtained by appropriately combining the technical means disclosed in different embodiments and examples are also included in the technical scope of the present invention. In addition, all documents recited in the present description are incorporated herein by reference.

Unless otherwise defined, technical and scientific terms used in the present invention have the same meaning as commonly understood by those of ordinary skill in the technical field to which the present invention belongs.

In the present description, a numerical range represented by "numerical value A to numerical value B" or "numerical value A-numerical value B" means a range including endpoint values A and B.

In the present description, where "%" appears, it means mass or weight percentage, *i.e.*, "% by mass" or "% by weight", unless otherwise specifically stated.

In the present description, the "normal temperature" or "room temperature" means an ambient temperature of 25°C.

In the present description, the meaning represented by "may" includes both the meaning of carrying out certain treatment and the meaning of not carrying out certain treatment. In the present description, the "optional" or "optionally" means that the subsequently described event or circumstance may or may not occur, and that the description includes instances where the event occurs and instances where it does not.

In the present description, reference to "some particular/preferred embodiments", "other particular/preferred embodiments", "some particular/preferred technical solutions", "other particular/preferred technical solutions" and the like mean that a particular element (e.g., feature, structure, property, and/or characteristic) described in connection with the embodiment is included in at least one embodiment described herein, and may or may not be present in other embodiments. In addition, it should be understood that the element can be combined in any suitable manner in various embodiments.

The term "comprise" and any variations thereof in the description and claims of the present invention are intended to cover non-exclusive inclusion.

In the present invention, the "pharmaceutically acceptable" means those that are, within the scope of reasonable medical judgment, suitable for use in contact with cells of humans and other animals without undue toxicity, irritation, allergic response and the like, and are commensurate with a reasonable benefit/risk ratio.

In the present invention, the "pharmaceutically acceptable salt" refers to a relatively non-toxic, inorganic and organic acid addition salt and alkali addition salt of the conjugate compound of the present application. Representative acid addition salts include hydrobromides, hydrochlorides, sulfates, bisulfates, phosphates, nitrates, acetates, oxalates, valerates, oleates, palmitates, stearates, laurates, borates, benzoates, lactates, tosylates, citrates, maleates, fumarates, succinates, tartrates, naphthoates, mesylates, glucoheptonates, lactiobionates, sulphamates, malonates, salicylates, propionates, methylene-bis-b-hydroxynaphthoates, gentisates, isethionates, di-p-toluoyltartrates, methanesulfonates, ethanesulphonates, benzenesulphonates, p-toluenesulphonates, cyclohexylsulphamates, quinateslaurylsulphonate, *etc.* Alkali addition salts include pharmaceutically acceptable metal and amine salts. Suitable metal salts include sodium, potassium, calcium, barium, zinc, magnesium, and aluminum salts. In some embodiments, sodium and potassium salts are preferred. Suitable inorganic alkali addition salts are prepared from base of metal which include, for example, sodium hydride, sodium hydroxide, potassium hydroxide, calcium hydroxide, aluminum hydroxide, lithium hydroxide, magnesium hydroxide, and zinc hydroxide. Suitable amine base addition salts are prepared from sufficiently basic amines to form stable salts, and preferably include the following common amines in pharmaceutical chemistry due to their low toxicity and acceptability for medical use: ammonia, ethylenediamine, N-methylglucamine, lysine, arginine, ornithine, choline, N,N'-dibenzylethylenediamine, chloroprocaine, diethanolamine, procaine, N-benzylphenethylamine, diethylamine, piperazine, tris(hydroxymethyl)aminomethane, tetramethylammonium hydroxide, triethylamine, dibenzylamine, diphenylhydroxylmethylamine, dehydroabietylamine, N-ethylpiperidine, benzylamine, tetramethylammonium, tetraethylammonium, methylamine, dimethylamine, trimethylamine, ethylamine, alkaline amino acids (e.g., lysine and arginine), dicyclohexylamine, *etc.*

In the present invention, the "ligand-drug conjugate (LDC)" is a product in which a biologically active drug is linked to a ligand via a chemical linker, wherein the ligand acts as a carrier to transport a small molecule drug to a cell of interest in a targeted manner, the ligand may be a polypeptide or a small molecule. Preferably, the ligand-drug conjugate is a dual ligand-drug conjugate, preferably, the dual ligand-drug conjugate is a drug conjugate targeting a folate receptor and a TRPV6 receptor. More preferably, the ligand-drug conjugate of the present invention is the ligand-drug conjugate recited in the patent application no. 201680045855.3 entitled "MULTI-LIGAND DRUG CONJUGATE AND USE THEREOF", more preferably, the ligand conjugate compound is dual ligand conjugate LDC10B, the ligands target a TRPV6 receptor and a folate receptor respectively, the loaded drug is MMAE, and the structure of the ligand conjugate compound is as follows: 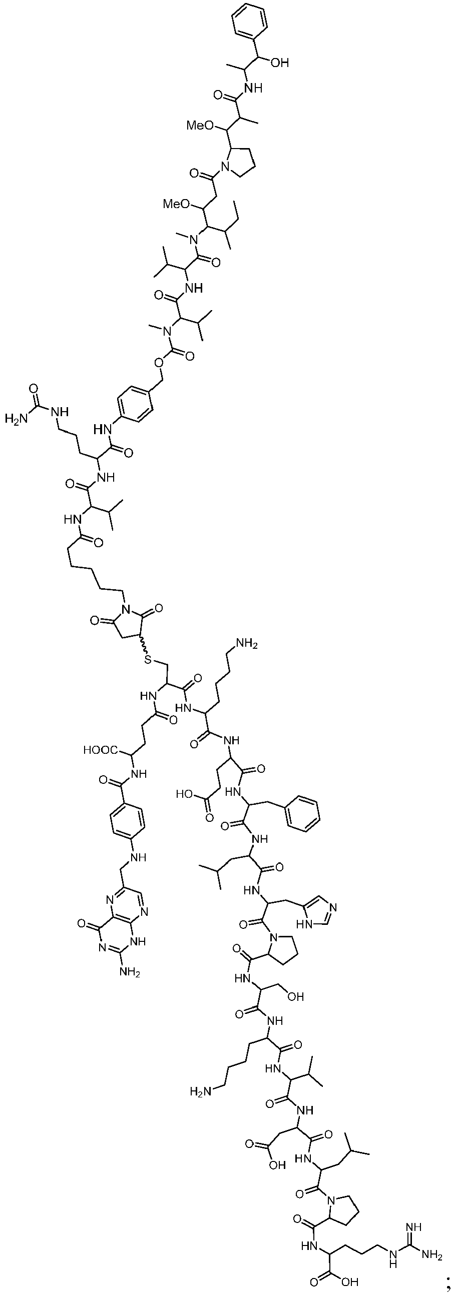 and further preferably, the dual ligand-drug conjugate has the following structure:

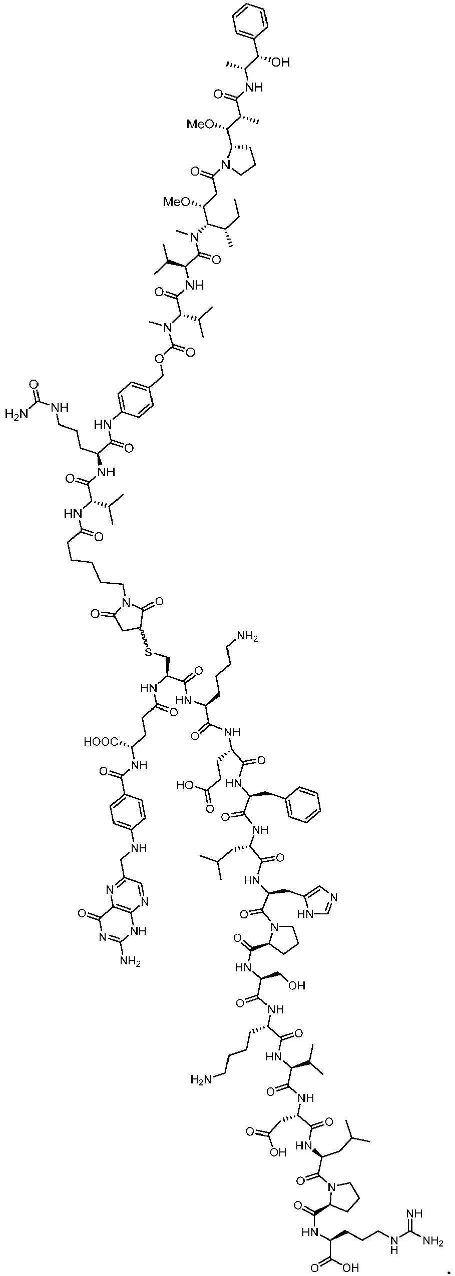

In the present invention, the "buffering agent" is a reagent that can maintain the pH value of a solution in an acceptable range. In some embodiments, the buffering agent used in the preparation of the present invention may control the pH value of the preparation of the present invention in a pH range of about 6.5-8.5, such as a pH value of about 6.9 to 8.0. In some particular embodiments, the preparation of the present invention has a pH value of about 6.9, 7.0, 7.1, 7.2, 7.3, 7.4, 7.5, 7.6, 7.7, 7.8, 7.9, or 8.0.

In the present invention, the "polyol" refers to a broad class of alcohols containing two or more hydroxyl groups in the molecule.

In the present invention, the "reconstitution" refers to dissolving and/or suspending a solid preparation (e.g., a freeze-dried preparation) in a physiologically acceptable solution.

In the present invention, the "about" means that the defined numerical value is approximate and that the particular numerical value in the relevant data needs not be very precise.

In the present invention, the "preparation" refers to a drug that is made in accordance with certain dosage form requirements and that can be finally provided for use by a subject.

In the present invention, the "freeze-dried preparation" means a sterile solid for injection prepared by a freeze-drying method.

In the present invention, the "lyophilized excipient", also known as a freeze-dried protectant, refers to an additive added during freeze-drying and storage of foods, drugs and organisms in order to keep the stability and activity of the product under the influence of many factors (such as chemical component, freezing rate, freezing and dehydration stress, glass transition temperature, residual moisture in the dried solid, and temperature and humidity of the storage environment).

In the present invention, the "stable preparation" means that the water content, appearance, pH value, impurity content, character after re-dissolution, *etc.* of a product remain substantially unchanged or change within pharmaceutically acceptable ranges during the preparation and storage.

In the present invention, the "HPLC" refers to high performance liquid chromatography, which is an important branch of chromatography. It uses liquid as the mobile phase, and uses a high-pressure injection system to pump the mobile phase such as single solvents with different polarities or solvents with different mixing ratios and buffers into the chromatographic column containing the stationary phase. After the components in the column are separated, they enter the detector for detection, so as to realize the analysis of the sample.

### [Pharmaceutical preparation comprising LDC and preparation method therefor]

The present invention provides a pharmaceutical preparation, which may comprise an active drug and optionally a pharmaceutically acceptable adjuvant.

In one embodiment, the above-mentioned pharmaceutical preparation may comprise a ligand-drug conjugate, a lyophilized excipient and a buffering agent.

In one embodiment, the above-mentioned pharmaceutical preparation may comprise a ligand-drug conjugate, a lyophilized excipient, a buffering agent and a solvent.

In one preferred embodiment, the ligand-drug conjugate in the above-mentioned pharmaceutical preparation may be a dual ligand-drug conjugate targeting a folate receptor and a TRPV6 receptor.

In one embodiment, the solvent in the above-mentioned pharmaceutical preparation is water.

In one preferred embodiment, the solvent in the above-mentioned pharmaceutical preparation is purified water.

In another preferred embodiment, the solvent in the above-mentioned pharmaceutical preparation is sterile water, distilled water, or deionized water.

In one more preferred embodiment, the solvent in the above-mentioned pharmaceutical preparation is sterile water for injection, single-distilled water, or double-distilled water.

In one embodiment, the active drug in the above-mentioned pharmaceutical preparation has a concentration of 4.75 mg/mL to 6 mg/mL, such as 4.75 mg/mL, 5 mg/mL, 5.25 mg/mL, 5.5 mg/mL, 5.75 mg/mL, or 6 mg/mL.

In one preferred embodiment, the active drug in the above-mentioned pharmaceutical preparation has a concentration of 5 mg/mL.

In one embodiment, the above-mentioned pharmaceutical preparation has a pH value of 6.5 to 8.5.

In one preferred embodiment, the above-mentioned pharmaceutical preparation may have a pH value of 6.9 to 8.0, such as 6.9, 7.0, 7.1, 7.2, 7.3, 7.4, 7.5, 7.6, 7.7, 7.8, 7.9, or 8.0.

In one more preferred embodiment, the above-mentioned pharmaceutical preparation may have a pH value of 6.9 to 7.7, such as 6.9, 7.0, 7.3, 7.5, or 7.7.

In one embodiment, the lyophilized excipient in the above-mentioned pharmaceutical preparation may comprise a polyol, such as one or more of mannitol, sorbitol, inositol, and xylitol.

In one preferred embodiment, the lyophilized excipient in the above-mentioned pharmaceutical preparation comprises mannitol.

In one embodiment, the lyophilized excipient in the above-mentioned pharmaceutical preparation comprises a polyol and saccharides, such as one or more of a monosaccharide, a disaccharide, and a polysaccharide.

Further, the monosaccharide in the lyophilized excipient in the above-mentioned pharmaceutical preparation comprises one or more of glucose and fructose; the disaccharide comprises one or more of sucrose, trehalose, maltose and lactose; and the polysaccharide comprises one or more of cyclodextrin and dextran.

Further, the mass ratio of the saccharides to the polyol is 1:1 to 1:5; preferably, the mass ratio of the saccharides to the polyol is 1:2 to 1:4.

In one preferred embodiment, the lyophilized excipient in the above-mentioned pharmaceutical preparation may comprise mannitol and sucrose, preferably, the mass ratio of the sucrose to the mannitol is 1:2 to 1:5, such as 1:2, 1:2.4, 1:2.5, 1:4, 1:4.5, 1:4.7, or 1:5.

In one more preferred embodiment, the lyophilized excipient in the above-mentioned pharmaceutical preparation may comprise mannitol and sucrose, preferably, the mass ratio of the sucrose to the mannitol is 1:4.

In another preferred embodiment, the lyophilized excipient in the above-mentioned pharmaceutical preparation may comprise mannitol and trehalose, preferably, the mass ratio of the trehalose to the mannitol is 1:2 to 1:4.

In another more preferred embodiment, the lyophilized excipient in the above-mentioned pharmaceutical preparation may comprise mannitol and trehalose, preferably, the mass ratio of the trehalose to the mannitol is 1:4.

In one embodiment, the buffering agent in the above-mentioned pharmaceutical preparation may comprise tromethamine and an acidic substance, the mass ratio of the tromethamine to the active drug is 1:4 to 1:10, preferably 1:5 to 1:8.5.

Further, the acidic substance comprised in the buffering agent in the above-mentioned pharmaceutical preparation may be one or more selected from hydrochloric acid, acetic acid, sodium dihydrogen phosphate and tromethamine hydrochloride.

In one preferred embodiment, the buffering agent in the above-mentioned pharmaceutical preparation may comprise tromethamine and hydrochloric acid, preferably, the mass ratio of the tromethamine to the active drug is 1:5 to 1:8.5, such as 1:5, 1:5.5, 1:6, 1:6.5, 1:7, 1:8, or 1:8.5.

In one more preferred embodiment, the buffering agent in the above-mentioned pharmaceutical preparation may comprise tromethamine and hydrochloric acid, preferably, the ratio of the tromethamine to the active drug is 1:6.5.

In another preferred embodiment, the buffering agent in the above-mentioned pharmaceutical preparation may comprise tromethamine and acetic acid, the ratio of the tromethamine to the active drug is 1:6.5.

In still another preferred embodiment, the buffering agent in the above-mentioned pharmaceutical preparation may comprise tromethamine and sodium dihydrogen phosphate, the ratio of the tromethamine to the active drug is 1:6.5.

In still another preferred embodiment, the buffering agent in the above-mentioned pharmaceutical preparation may comprise tromethamine and tromethamine hydrochloride, the ratio of the tromethamine to the active drug is 1:6.5.

In one embodiment, each 1 mL of the above-mentioned pharmaceutical preparation comprises 4.75-6 mg of a ligand-drug conjugate or a pharmaceutically acceptable salt thereof, 25-55 mg of mannitol, 0-55 mg of sucrose, 0.5-1.25 mg of tromethamine, hydrochloric acid, and the balance made up of a solvent; wherein the pharmaceutical preparation has a pH value of 6.5-8.5, preferably 6.9 to 8.0.

In one preferred embodiment, each 1 mL of the above-mentioned pharmaceutical preparation comprises 5 mg of a ligand-drug conjugate or a pharmaceutically acceptable salt thereof, 40 mg of mannitol, 10 mg of sucrose, 0.765 mg of tromethamine, hydrochloric acid, and the balance made up of water; wherein the pharmaceutical preparation has a pH value of 6.5-8.5, preferably 6.9 to 8.0.

In one embodiment, the above-mentioned pharmaceutical preparation is sterile.

In one embodiment, the above-mentioned pharmaceutical preparation is stable during freezing and thawing.

In one embodiment, a preparation method for preparing the above-mentioned pharmaceutical preparation comprises the following steps: using a prescription amount of an active drug and optionally adding a pharmaceutically acceptable adjuvant, and carrying out uniform mixing to obtain the pharmaceutical preparation.

In one preferred embodiment, a preparation method for preparing the above-mentioned pharmaceutical preparation comprises the following steps: using a prescription amount of a ligand-drug conjugate or a pharmaceutically acceptable salt thereof, a lyophilized excipient, a buffering agent and a solvent, dissolving the ligand-drug conjugate or the pharmaceutically acceptable salt thereof and the lyophilized excipient using the solvent, adjusting the pH value to 6.9 to 7.7 using the buffering agent, carrying out uniform mixing, and carrying out filtering to obtain the pharmaceutical preparation.

### [Freeze-dried preparation and preparation method therefor]

The present invention provides a freeze-dried preparation prepared by freeze-drying the pharmaceutical preparation of the present invention.

The present invention further provides a method for preparing the above-mentioned freeze-dried preparation, comprising the following steps: freeze-drying the pharmaceutical preparation of the present invention to obtain the freeze-dried preparation.

In one embodiment, in the above-mentioned preparation method, the freeze-drying comprises the following steps: freezing the pharmaceutical preparation at -45°C; annealing the pharmaceutical preparation at -25°C; re-freezing the pharmaceutical preparation at -45°C; carrying out first drying on the pharmaceutical preparation at - 15°C; and carrying out second drying on the pharmaceutical preparation at 25°C.

In one preferred embodiment, in the above-mentioned preparation method, the freeze-drying comprises the following steps: freezing the pharmaceutical preparation at -45°C; annealing the pharmaceutical preparation at -25°C; re-freezing the pharmaceutical preparation at -45°C; carrying out first drying on the pharmaceutical preparation at -15°C for 800 to 3000 min; and carrying out second drying on the pharmaceutical preparation at 25°C for 300 to 600 min.

In one more preferred embodiment, in the above-mentioned preparation method, the freeze-drying comprises the following steps: freezing: placing the prescription solution in a freeze dryer, and at atmospheric pressure, carrying out cooling to -45°C and keeping the temperature for 120 min; annealing: at atmospheric pressure, carrying out heating to -25°C and keeping the temperature for 180 min; freezing: at atmospheric pressure, carrying out cooling to -45°C and keeping the temperature for 120 min; pre-vacuumizing: changing the atmospheric pressure in the freeze dryer to 0.2 mbar; first drying: at 0.13 mbar, carrying out heating to -15°C and keeping the temperature for 800 to 3000 min; second drying: at 0.13 mbar, carrying out heating to 25°C and keeping the temperature for 300 to 600 min.

In one more preferred embodiment, in the above-mentioned preparation method, the freeze-drying comprises the following steps: freezing: placing the prescription solution in a freeze dryer, and at atmospheric pressure, carrying out cooling to -45°C and keeping the temperature for 120 min; annealing: at atmospheric pressure, carrying out heating to -25°C and keeping the temperature for 180 min; freezing: at atmospheric pressure, carrying out cooling to -45°C and keeping the temperature for 120 min; pre-vacuumizing: changing the atmospheric pressure in the freeze dryer to 0.2 mbar; first drying: at 0.13 mbar, carrying out heating to -15°C and keeping the temperature for 800 min; second drying: at 0.13 mbar, carrying out heating to 25°C and keeping the temperature for 600 min.

### [Liquid preparation and preparation method therefor]

The present invention provides a liquid preparation reconstituted by the freeze-dried preparation of the present invention using water.

The present invention further provides a preparation method for preparing the above-mentioned liquid preparation, comprising the following steps: mixing the freeze-dried preparation of the present invention with water to obtain the liquid preparation.

In one embodiment, in the above-mentioned preparation method, the reconstitution is carried out using distilled water, pure water, or sterile water.

In one embodiment, the freeze-dried preparation has a pH value of 6.5 to 8.5 after being reconstituted using water.

### [Device]

The present invention provides a drug-containing delivery device, comprising one of the above-mentioned pharmaceutical preparation, the above-mentioned freeze-dried preparation, or the above-mentioned liquid preparation.

The present invention further provides a pre-filled syringe, comprising one of the above-mentioned pharmaceutical preparation, the above-mentioned freeze-dried preparation, or the above-mentioned liquid preparation.

### [Medical use]

The present invention provides the use of the above-mentioned pharmaceutical preparation comprising an active drug, the above-mentioned freeze-dried preparation, or the above-mentioned liquid preparation in the preparation of a delivery device or a pre-filled syringe or a drug.

In one embodiment, the delivery device or pre-filled syringe or drug in the above-mentioned use is used for enhancing an immune effector cell response in a subject.

In one embodiment, the delivery device or pre-filled syringe or drug in the above-mentioned use is used for reducing immunosuppression in a subject.

In one embodiment, the delivery device or pre-filled syringe or drug in the above-mentioned use is used for the treatment or prevention of a cancer in a subject.

Further, the cancer in the above-mentioned use comprises one or more of breast cancer, lung cancer, prostate cancer, kidney cancer, leukemia, ovarian cancer, stomach cancer, uterine cancer, endometrial cancer, liver cancer, colon cancer, thyroid cancer, pancreatic cancer, colorectal cancer, esophageal cancer, skin cancer, lymphoma, and multiple myeloma.

In one embodiment, the delivery device or pre-filled syringe or drug in the above-mentioned use is used for the treatment or prevention of an immune disease in a subject.

Further, the immune disease in the above-mentioned use comprises one or more of a connective tissue disease, systemic sclerosis, rheumatoid arthritis, and systemic lupus erythematosus.

In one embodiment, the delivery device or pre-filled syringe or drug in the above-mentioned use is used for the treatment or prevention of a cardiovascular disease in a subject.

Further, the cardiovascular disease in the above-mentioned use comprises one or more of angina pectoris, myocardial infarction, apoplexy, heart attack, a hypertensive heart disease, a rheumatic heart disease, cardiomyopathy, cardiac arrhythmia, and a congenital heart disease.

In one embodiment, the delivery device or pre-filled syringe or drug in the above-mentioned use is used for the treatment or prevention of a metabolic disease in a subject.

Further, the metabolic disease in the above-mentioned use comprises one or more of diabetes mellitus, gout, obesity, hypoglycemia, hyperglycemia, and dyslipidemia.

In one embodiment, the delivery device or pre-filled syringe or drug in the above-mentioned use is used for the treatment or prevention of a neurological disease in a subject.

Further, the neurological disease in the above-mentioned use comprises one or more of Alzheimer's disease, Parkinson's disease, Huntington's disease, head injury, multiple sclerosis, vertigo, coma, and epilepsy.

Unless otherwise stated, instrument devices, reagents, materials, *etc.* used in the present invention can be acquired by conventional commercial means. The technical solutions of the present invention are further described below in conjunction with particular examples.

### Example 1

To investigate the stability of the preparation comprising LDC10B at pH 6.9 to 8.0, a total of 5 pH values, namely 6.9, 7.2, 7.5, 7.8 and 8.0, were designed.

### 1.1 Experimental steps

An aqueous solution containing LDC10B (5 mg/mL), mannitol (40 mg/mL), sucrose (10 mg/mL) and tromethamine (0.765 mg/mL) was prepared, and the pH was adjusted to 6.9, 7.2, 7.5, 7.8 and 8.0 with dilute hydrochloric acid, respectively; the resulting solutions were filtered and sub-packaged into vials, and the vials were plugged and capped. The stability of each sample was investigated at room temperature for 12 h.

### 1.2 Experimental scheme

The specific scheme is as shown in the table below:

| **Condition** | **Sampling point** | | | **Detection items** |
|---|---|---|---|---|
| Room temperature | 0h | 4h | 12h | Related substances, solution appearance |

### 1.3 Experimental results

| **pH value** | **Condition** | **Amount of impurity** | **% Total impurity** | **Appearance** |
|---|---|---|---|---|
| 8.0 | 0h | 14 | 1.47 | Yellow clarified solution |
| | 4h | 16 | 2.68 | Yellow clarified solution |
| | 12h | 18 | 2.75 | Yellow clarified solution |
| 7.8 | 0h | 15 | 1.48 | Yellow clarified solution |
| | 4h | 16 | 1.81 | Yellow clarified solution |
| | 12h | 18 | 2.16 | Yellow clarified solution |
| 7.5 | 0h | 14 | 1.23 | Yellow clarified solution |
| | 4h | 15 | 1.54 | Yellow clarified solution |
| | 12h | 17 | 1.81 | Yellow clarified solution |
| 7.2 | 0h | 14 | 1.24 | Yellow clarified solution |
| | 4h | 15 | 1.33 | Yellow clarified solution |
| | 12h | 16 | 1.61 | Yellow clarified solution |
| 6.9 | 0h | 13 | 1.27 | Yellow clarified solution |
| | 4h | 15 | 1.34 | Yellow clarified solution |
| | 12h | 15 | 1.44 | Yellow clarified solution |

After placing at pH 6.9, 7.2, 7.5, 7.8 and 8.0 for different times at room temperature, the related substances of each sample were determined by HPLC. It can be seen from the investigation results that the total impurities in the samples placed for 12 h at room temperature at each pH value all increased, and compared with the related substances in the sample placed for 0 h, the total impurities in the samples at pH 6.9, 7.2, 7.5, 7.8 and 8.0 increased by 0.17%, 0.37%, 0.58%, 0.68% and 1.28%, respectively.

The above-mentioned experimental results showed that at a pH between 6.9 to 8.0, the total impurities were all within acceptable ranges.

### 1.4 Preparation of sample into final preparation form (freeze-dried form) for pH confirmation

### 1.4.1 Experimental steps

An aqueous solution containing LDC10B (5 mg/mL), mannitol (40 mg/mL), sucrose (10 mg/mL) and tromethamine (0.765 mg/mL) was prepared, and the pH was adjusted to 6.9, 7.3 and 7.7 with dilute hydrochloric acid, respectively; the resulting solutions were filtered and sub-packaged into vials, half of a stopper was pressed into the vial, freeze-drying was performed, and the vials were capped. The stability of each sample was investigated at 30°C for 1 month.

### 1.4.2 Experimental scheme

The specific scheme is as shown in the table below:

| **Condition** | **Sampling point** | | **Detection items** |
|---|---|---|---|
| 30°C | 0 day | 30 day | Related substances, appearance |

### 1.4.3 Experimental results

| **Prescription pH** | **% Total impurity** | | **Appearance** | |
|---|---|---|---|---|
| | 0 day | 30 day | 0 day | 30 day |
| pH 6.9 | 0.40 | 0.41 | Slightly yellow block | Slightly yellow block |
| pH 7.3 | 0.59 | 0.40 | Slightly yellow block | Slightly yellow block |
| pH 7.7 | 0.49 | 0.43 | Slightly yellow block | Slightly yellow block |

The experimental results were as shown in the above table. The data showed that the quality of the freeze-dried preparation containing LDC10B was stable in the pH range of 6.9 to 7.7.

### Example 2

To study the effect of a tromethamine buffer system, a phosphate buffer system and an acetate buffer system on the stability of the preparation containing LDC10B, a total of 4 prescriptions were designed. The detailed prescription information is as shown in the table below:

| **Prescription 1** | **Prescription 2** | **Prescription 3** | **Prescription 4** |
|---|---|---|---|
| LDC10B 10 mg | LDC10B 10 mg | LDC10B 10 mg | LDC10B 10 mg |
| 1 M acetic acid Q.S. | 0.1 M hydrochloric acid Q.S. | Sodium dihydrogen phosphate 0.92 mg | Tromethamine hydrochloride 12.61 mg |
| Tromethamine 1.53 mg | Tromethamine 1.53 mg | Tromethamine 1.53 mg | Tromethamine 2.42 mg |
| Mannitol 80.00 mg | Mannitol 80.00 mg | Mannitol 80.00 mg | Mannitol 80.00 mg |
| Sucrose 20.00 mg | Sucrose 20.00 mg | Sucrose 20.00 mg | Sucrose 20.00 mg |
| Water to 2 mL | Water to 2 mL | Water to 2 mL | Water to 2 mL |
| pH 7.41 | pH 7.30 | pH 7.44 | pH 7.44 |

### 2.1 Experimental steps

Aqueous solutions of each of the above-mentioned prescriptions were prepared, and filtered and sub-packaged into vials, half of a stopper was pressed into the vial, freeze-drying was performed, and the vials were capped. The stability of each sample was investigated at 40°C.

### 2.2 Experimental scheme

The specific scheme is as shown in the table below, and the detection indicators are appearance and related substances.

| **Condition** | **Sampling point** | | **Detection items** |
|---|---|---|---|
| 40°C | 0 day | 10 day | Related substances, appearance |

### 2.3 Experimental results

| **Prescription** | **Appearance** | | **% Total impurity** | |
|---|---|---|---|---|
| | 0 day | 40°C for 10 days | 0 day | 40°C for 10 days |
| Prescription 1 | Slightly yellow block | Slightly yellow block | 0.78 | 0.93 |
| Prescription 2 | Slightly yellow block | Slightly yellow block | 0.59 | 0.57 |
| Prescription 3 | Slightly yellow block | Slightly yellow block | 0.93 | 1.08 |
| Prescription 4 | Slightly yellow block | Slightly yellow block | 1.06 | 1.09 |

The above-mentioned experimental results showed that the changes in the total impurity of each prescription were all within acceptable ranges at 40°C.

### Example 3

Under the buffer system obtained, the amount of tromethamine (0.615 to 1.00 mg/mL) was screened and optimized to obtain better product quality.

| **Prescription 5** | **Prescription 6** | **Prescription 7** | **Prescription 8** |
|---|---|---|---|
| LDC10B 10 mg | LDC10B 10 mg | LDC10B 10 mg | LDC10B 10 mg |
| 0.1 M hydrochloric acid Q.S. | 0.1 M hydrochloric acid Q.S. | 0.1 M hydrochloric acid Q.S. | 0.1 M hydrochloric acid Q.S. |
| Tromethamine 1.23 mg | Tromethamine 1.43 mg | Tromethamine 1.53 mg | Tromethamine 2.00 mg |
| Mannitol 80.00 mg | Mannitol 80.00 mg | Mannitol 80.00 mg | Mannitol 80.00 mg |
| Sucrose 20.00 mg | Sucrose 20.00 mg | Sucrose 20.00 mg | Sucrose 20.00 mg |
| Water to 2 mL | Water to 2 mL | Water to 2 mL | Water to 2 mL |

### 3.1 Experimental steps

Aqueous solutions of each of the above-mentioned prescriptions were prepared, and filtered and sub-packaged into vials, half of a stopper was pressed into the vial, freeze-drying was performed, and the vials were capped. The stability of each sample was investigated at 2°C to 8°C.

### 3.2 Experimental results

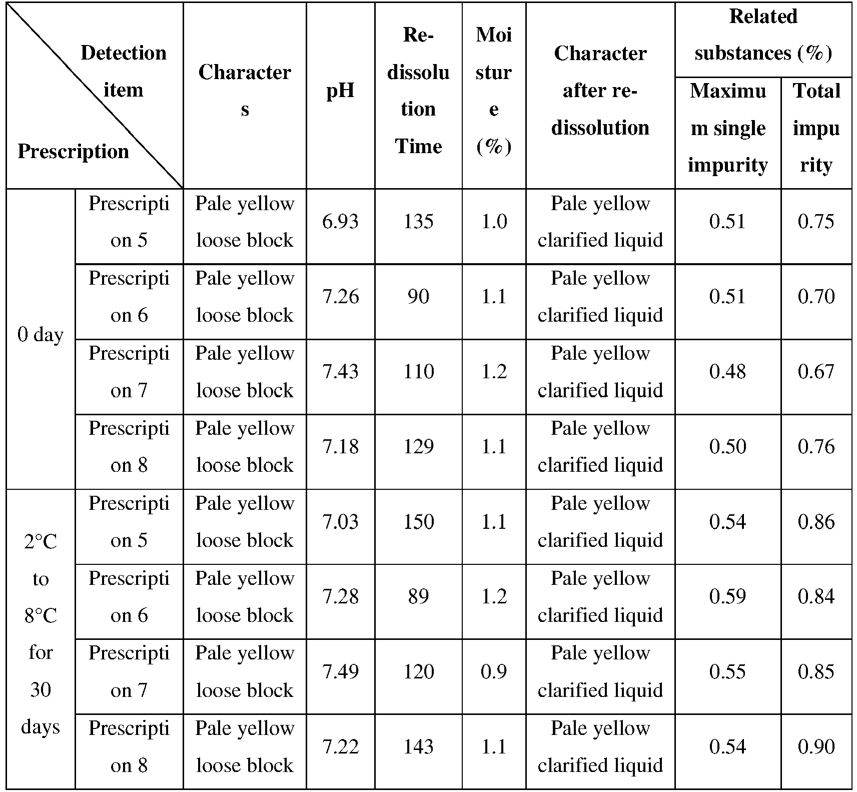

The results showed that there was no significant difference in the re-dissolution time, characters, moisture and related substances of the products when the amount of tromethamine ranged from 0.615 mg/mL to 1.00 mg/mL, indicating that 0.615 mg/mL to 1.00 mg/mL was an acceptable amount range.

### Example 4

To study the effect of mannitol as the polyol and sucrose and trehalose as the saccharides on the stability of the preparation containing LDC10B, a total of 4 prescriptions were designed. The detailed prescription information is as shown in the table below:

| **Prescription 9** | **Prescription 10** | **Prescription 11** | **Prescription 12** |
|---|---|---|---|
| LDC10B 10 mg | LDC10B 10 mg | LDC10B 10 mg | LDC10B 10 mg |
| 0.1 M hydrochloric acid Q.S. | 0.1 M hydrochloric acid Q.S. | 0.1 M hydrochloric acid Q.S. | 0.1 M hydrochloric acid Q.S. |
| Tromethamine 1.23 mg | Tromethamine 1.23 mg | Tromethamine 1.23 mg | Tromethamine 1.23 mg |
| Sucrose 100 mg | Trehalose 100 mg | Mannitol 80 mg | Mannitol 80 mg |
| / | / | Sucrose 20 mg | Trehalose 20 mg |
| Water to 2 mL | Water to 2 mL | Water to 2 mL | Water to 2 mL |

### 4.1 Experimental steps

Aqueous solutions of each of the above-mentioned prescriptions were prepared, and filtered and sub-packaged into vials, half of a stopper was pressed into the vial, freeze-drying was performed, and the vials were capped. After freeze-drying, the products of prescription 9 and prescription 10 taken out of the freeze dryer shrunk, while the products of the remaining prescriptions did not have this phenomenon.

### 4.2 Experimental scheme

The specific scheme is as shown in the table below, and the detection indicators are appearance, moisture and related substances.

| **Condition** | **Sampling point** | **Detection items** |
|---|---|---|
| 2°C-8°C | 0 day, 30 days | Appearance, moisture, related substances |

### 4.3 Experimental results

| **Prescription** | **Appearance** | | **% Moisture** | | **% Total impurity** | |
|---|---|---|---|---|---|---|
| | 0 day | 2°C-8°C for 30 days | 0 day | 2°C-8°C for 30 days | 0 day | 2°C-8°C for 30 days |
| Prescription 9 | Shrunk | Shrunk | 0.9 | 1.2 | NA | NA |
| Prescription 10 | Shrunk | Shrunk | 1.4 | 1.3 | NA | NA |
| Prescription 11 | Slightly yellow block | Slightly yellow block | 1.5 | 1.1 | 0.74 | 0.60 |
| Prescription 12 | Slightly yellow block | Slightly yellow block | 1.3 | 1.2 | 0.72 | 0.73 |

| | | | | | | |
|---|---|---|---|---|---|---|
| Notes: "NA" stands for no relevant data. | | | | | | |

The above experimental results showed that the products shrunk when sucrose (prescription 9) and trehalose (prescription 10) were used as fillers, and the freeze-dried preparation with qualified appearance, moisture and quality could be obtained after mannitol is added. Therefore, mannitol and sucrose or mannitol and trehalose could be selected as excipients.

### Example 5

Under the excipient system obtained, the amounts of mannitol and sucrose were screened and optimized to obtain better product quality.

| **Material** | **Prescription 13** | **Prescription 14** | **Prescription 15** | **Prescription 16** | **Prescription 17** |
|---|---|---|---|---|---|
| **LDC10B** | 10.00 mg | 10.00 mg | 10.00 mg | 10.00 mg | 10.00 mg |
| **Hydrochloric acid** | Q.S. | Q.S. | Q.S. | Q.S. | Q.S. |
| **Tromethamine** | 1.53 mg | 1.53 mg | 1.53 mg | 1.53 mg | 1.53 mg |
| **Mannitol** | 80 mg | 80 mg | 93.5 mg | 80.0 mg | 90.8 mg |
| **Sucrose** | 0 | 20 mg | 20.0 mg | 33.5 mg | 22.7 mg |
| **Water** | To 2 mL | To 2 mL | To 2 mL | To 2 mL | To 2 mL |

### 5.1 Experimental steps

Aqueous solutions of each of the above-mentioned prescriptions were prepared, and filtered and sub-packaged into vials, half of a stopper was pressed into the vial, freeze-drying was performed, and the vials were capped. The stability of each sample was investigated at 40°C.

### 5.2 Experimental results

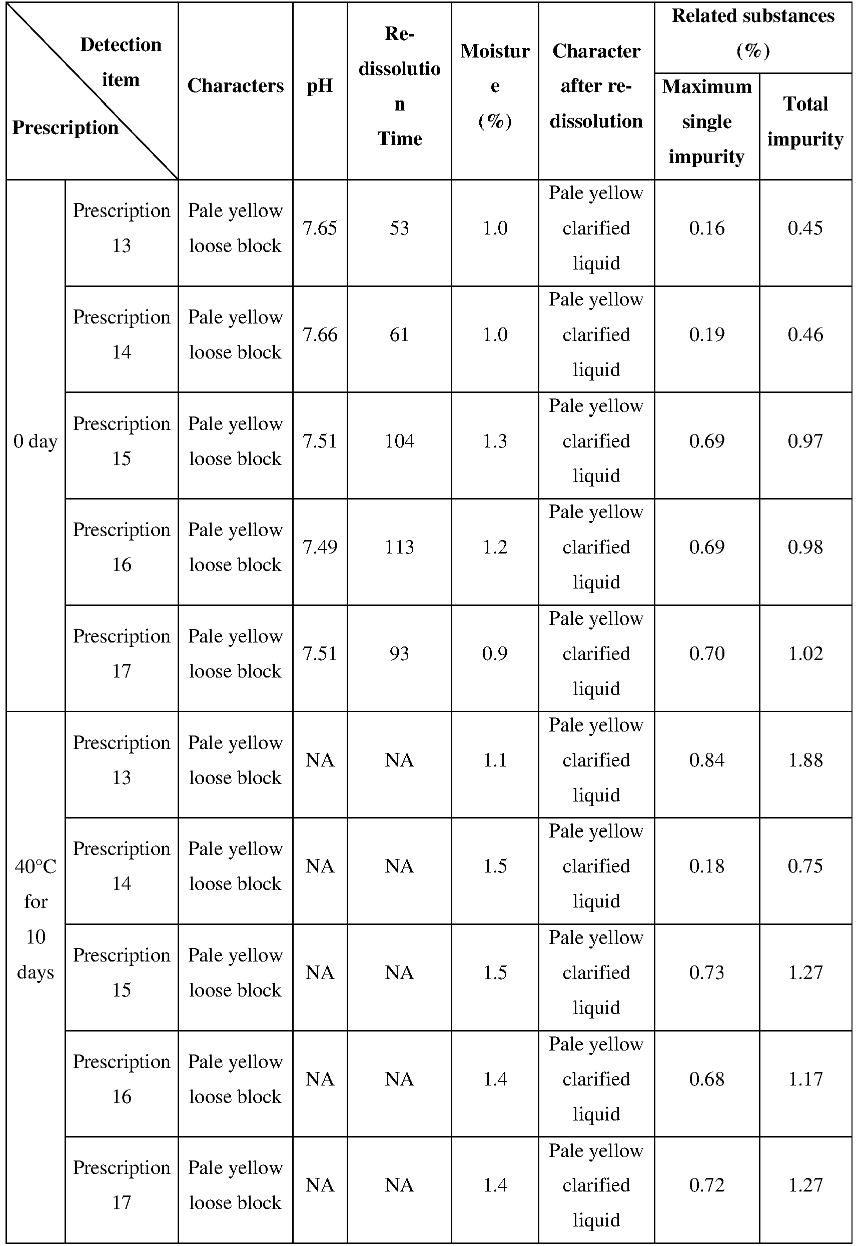

The results showed that there was a significant increase in the impurity of the product when mannitol was used alone, and a significant decrease in the amount of impurities when sucrose and mannitol were used together as excipients. Therefore, the amounts of excipients in the present product were as follows: sucrose 20 mg to 33.5 mg, mannitol 80 mg to 93.5 mg.

### Example 6

### 6.1 Selection of process

| **Procedure** | | **Shelve temperature (°C)** | **Temperature change rate (°C/min)** | **Heating time (min)** | **Holding time (min)** | **Pressure in freeze dryer (mbar)** |
|---|---|---|---|---|---|---|
| Freezing | | -45 | 1.00 | 50.00 | 120.00 | Atmospheric pressure |
| Annealing | | -25 | 1.00 | 20.00 | 180.00 | Atmospheric pressure |
| Freezing | | -45 | 1.00 | 20.00 | 120.00 | Atmospheric pressure |
| Pre-vacuumizing | | -45 | / | / | / | 0.2 |
| Process I | First drying | -15 | 0.20 | 150.00 | 3000.00 | 0.13 |
| | Second drying | 25 | 0.50 | 200.00 | 600.00 | 0.13 |
| Process II | First drying | -15 | 0.20 | 150.00 | 1200.00 | 0.13 |
| | Second drying | 25 | 0.50 | 200.00 | 600.00 | 0.13 |
| Process III | First drying | -15 | 0.20 | 150.00 | 800.00 | 0.13 |
| | Second drying | 25 | 0.50 | 200.00 | 600.00 | 0.13 |
| Process IV | First drying | -15 | 0.20 | 150.00 | 800.00 | 0.13 |
| | Second drying | 25 | 0.50 | 200.00 | 300.00 | 0.13 |
| Process V | First drying | -15 | 0.20 | 150.00 | 1200.00 | 0.13 |
| | Second drying | 25 | 0.50 | 200.00 | 300.00 | 0.13 |

### 6.2 Experimental results

| **Process** | **Appearance** | **% Moisture** | **Re-dissolution time s** | **pH value** |
|---|---|---|---|---|
| Process I | Slightly yellow block | 1.3% | 130 | 7.41 |
| Process II | Slightly yellow block | 1.0% | 85 | 7.45 |
| Process III | Slightly yellow block | 0.4% | 116 | 7.31 |
| Process IV | Slightly yellow block | 1.1% | 160 | 7.40 |
| Process V | Slightly yellow block | 1.0% | 91 | 7.35 |

All processes resulted in the products with good appearance, qualified moisture, and re-dissolution time and pH value that meet clinical use. The acceptable freeze-drying process was that the freezing was carried out at -45 °C and the annealing was carried out at -25°C; the first drying was carried out at -15°C for 800 min to 3000 min; the second drying was carried out at 25°C for 300 min to 600 min.

### Example 7

In view of the results of the prescription screening experiment, the prescription process confirmation experiment was carried out to investigate the stability of the final prescription.

The prescription information is as shown in the table below:

| **Name** | **Amount** | **Role** |
|---|---|---|
| LDC10B | 10 mg | Active component |
| Tromethamine | 1.53 mg | pH regulator |
| Mannitol | 80 mg | Lyophilized excipient |
| Sucrose | 20 mg | Lyophilized excipient |
| 0.1 M hydrochloric acid | Q.S. | pH regulator |
| Water | To 2 mL | Solvent |

The process information is as shown in the table below:

| **Procedure** | **Shelve temperature (°C)** | **Temperature change rate (°C/min)** | **Heating time (min)** | **Holding time (min)** | **Pressure in freeze dryer (mbar)** |
|---|---|---|---|---|---|
| Freezing | -45 | 1.00 | 50.00 | 120.00 | Atmospheric pressure |
| Annealing | -25 | 1.00 | 20.00 | 180.00 | Atmospheric pressure |
| Freezing | -45 | 1.00 | 20.00 | 120.00 | Atmospheric pressure |
| Pre-vacuumizing | -45 | / | / | / | 0.2 |
| First drying | -15 | 0.20 | 150.00 | 800.00 | 0.13 |
| Second drying | 25 | 0.50 | 200.00 | 600.00 | 0.13 |
| Total freeze-drying time | 37.7h | | | | |

The experimental scheme is as shown in the table below:

| **Condition** | **Sampling point** | **Detection items** |
|---|---|---|
| 25°C | 0 day, 1 month, 2 months, 3 months, 6 months | Appearance, moisture, re-dissolution pH, related substances |
| 40°C | 5 days, 10 day, 30 days | Appearance, moisture, re-dissolution pH, related substances |

The experimental results are as shown below:

| **Condition** | **Sampling point** | **% Moisture** | **% Total impurity** | **pH value** | **Appearance** | **Re-dissolution time s** |
|---|---|---|---|---|---|---|
| 25°C | 0 day | 0.42 | 0.44 | 7.4 | Slightly yellow block | 160 |
| | 1 month | 1.79 | 0.51 | 7.5 | Slightly yellow block | 154 |
| | 2 months | 1.97 | 0.61 | 7.2 | Slightly yellow block | 114 |
| | 3 months | 2.19 | 0.81 | 7.4 | Slightly yellow block | 174 |
| | 6 months | 2.62 | 1.21 | 7.3 | Slightly yellow block | 128 |
| 40°C | 0 day | 1.2 | 0.33 | 7.4 | Slightly yellow block | 148 |
| | 5 day | 1.0 | 0.48 | 7.1 | Slightly yellow block | 139 |
| | 10 day | 1.2 | 0.54 | 7.3 | Slightly yellow block | 118 |
| | 30 day | 1.8 | 0.69 | 7.4 | Slightly yellow block | 135 |

The results showed that under the conditions of 25°C-6 months and 40°C-1 month, the changes in the appearance, moisture (< 5%), re-dissolution pH (6.9 to 7.7) and related substances (total impurity < 5%) of the product prepared by the determined prescription and process were all within acceptable ranges.

## Claims

1. A pharmaceutical preparation, **characterized in that** the pharmaceutical preparation comprises an active drug and optionally a pharmaceutically acceptable adjuvant.

2. The pharmaceutical preparation according to claim 1, **characterized in that**
the active drug comprises a ligand-drug conjugate or a pharmaceutically acceptable salt thereof;
the pharmaceutically acceptable adjuvant comprises one or more of a lyophilized excipient and a buffering agent.

3. The pharmaceutical preparation according to claim 2, **characterized in that**
the active drug comprises a ligand-drug conjugate or a pharmaceutically acceptable salt thereof;
the pharmaceutically acceptable adjuvant comprises one or more of a lyophilized excipient, a buffering agent and a solvent.

4. The pharmaceutical preparation according to claim 2 or 3, **characterized in that**
the ligand-drug conjugate is a dual ligand-drug conjugate;
preferably, the dual ligand-drug conjugate is a drug conjugate targeting a folate receptor and a TRPV6 receptor;
more preferably, the dual ligand-drug conjugate has the following structure: 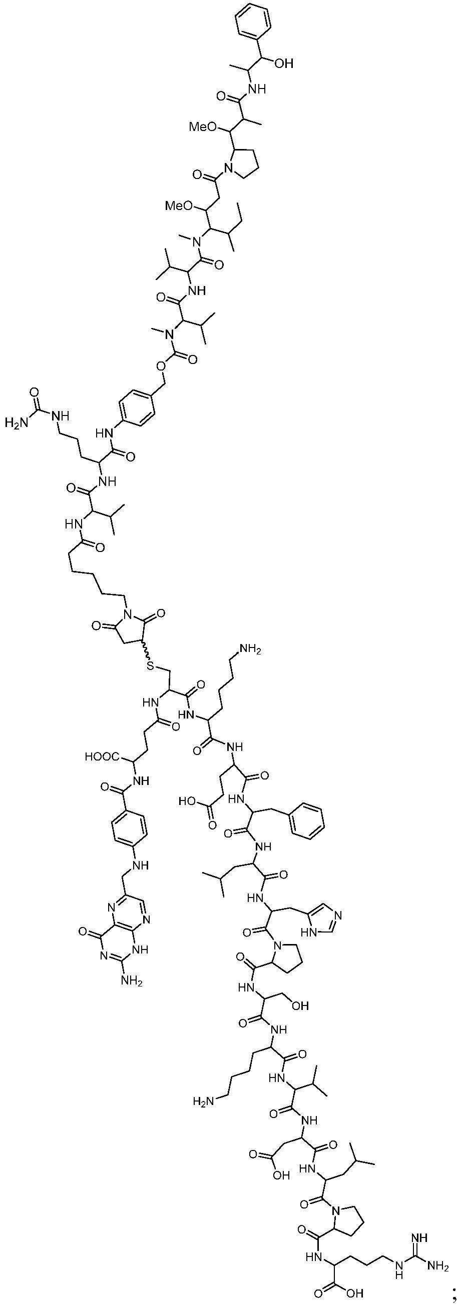
and further preferably, the dual ligand-drug conjugate has the following structure:

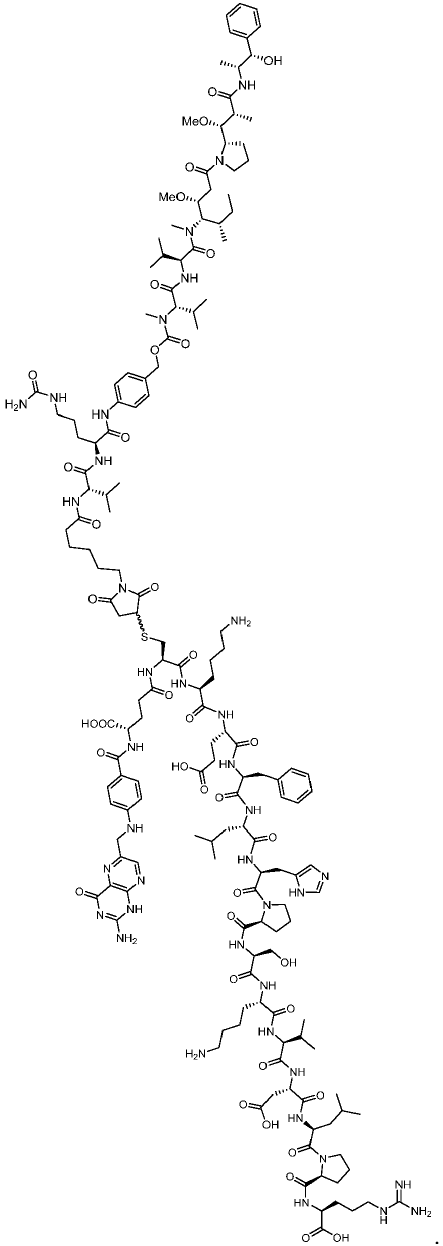

5. The pharmaceutical preparation according to claim 3 or 4, **characterized in that** the solvent is water;
preferably, the solvent is purified water;
more preferably, the purified water is sterile water, distilled water, or deionized water;
and more preferably, the sterile water is sterile water for injection, single-distilled water, or double-distilled water.

6. The pharmaceutical preparation according to claim 3 or 4, **characterized in that**
the active drug has a concentration of 4.75 mg/mL to 6 mg/mL;
and preferably, the active drug has a concentration of 4.75 mg/mL, 5 mg/mL, 5.25 mg/mL, 5.5 mg/mL, 5.75 mg/mL, or 6 mg/mL.

7. The pharmaceutical preparation according to any one of claims 3 to 6, **characterized in that**
the pharmaceutical preparation has a pH value of 6.5 to 8.5;
preferably, the pharmaceutical preparation has a pH value of 6.9 to 8.0, such as 6.9, 7.0, 7.1, 7.2, 7.3, 7.4, 7.5, 7.6, 7.7, 7.8, 7.9, or 8.0;
and more preferably, the pharmaceutical preparation has a pH value of 6.9 to 7.7, such as 6.9, 7.0, 7.3, 7.5, or 7.7.

8. The pharmaceutical preparation according to any one of claims 2 to 7, **characterized in that**
the lyophilized excipient comprises a polyol, such as one or more of mannitol, sorbitol, inositol, and xylitol;
and preferably, the lyophilized excipient comprises mannitol.

9. The pharmaceutical preparation according to claim 8, **characterized in that**
the lyophilized excipient further comprises a saccharide, such as one or more of a monosaccharide, a disaccharide, and a polysaccharide;
preferably, a mass ratio of the saccharides to the polyol is 1:1 to 1:5;
and more preferably, a mass ratio of the saccharide to the polyol is 1:2 to 1:4.

10. The pharmaceutical preparation according to claim 9, **characterized in that**
the monosaccharide comprises one or more of glucose and fructose;
the disaccharide comprises one or more of sucrose, trehalose, maltose, and lactose;
and the polysaccharide comprises one or more of cyclodextrin and dextran.

11. The pharmaceutical preparation according to any one of claims 8 to 10, **characterized in that** the lyophilized excipient comprises mannitol and sucrose; and preferably, a mass ratio of the sucrose to the mannitol is 1:2 to 1:5, such as 1:2, 1:2.4, 1:2.5, 1:4, 1:4.5, 1:4.7, or 1:5.

12. The pharmaceutical preparation according to any one of claims 8 to 10, **characterized in that** the lyophilized excipient comprises mannitol and trehalose; and preferably, a mass ratio of the trehalose to the mannitol is 1:2 to 1:4.

13. The pharmaceutical preparation according to claim 2 or 3, **characterized in that** the buffering agent comprises tromethamine and an acidic substance; and a mass ratio of the tromethamine to the active drug is 1:4 to 1:10, preferably 1:5 to 1:8.5.

14. The pharmaceutical preparation according to claim 13, **characterized in that** the acidic substance comprises one or more of hydrochloric acid, acetic acid, sodium dihydrogen phosphate, and tromethamine hydrochloride.

15. The pharmaceutical preparation according to claim 13 or 14, **characterized in that** the buffering agent comprises tromethamine and hydrochloric acid, preferably, a mass ratio of the tromethamine to the active drug is 1:5 to 1:8.5, such as 1:5, 1:5.5, 1:6, 1:6.5, 1:7, 1:8, or 1:8.5.

16. The pharmaceutical preparation according to claim 1, **characterized in that** each 1 mL of the pharmaceutical preparation comprises 4.75-6 mg of a ligand-drug conjugate or a pharmaceutically acceptable salt thereof, 25-55 mg of mannitol, 0-55 mg of sucrose, 0.5-1.25 mg of tromethamine, hydrochloric acid, and the balance made up of a solvent; wherein the pharmaceutical preparation has a pH value of 6.5 to 8.5, preferably 6.9 to 8.0;
for example, each 1 mL of the pharmaceutical preparation comprises 5 mg of a ligand-drug conjugate or a pharmaceutically acceptable salt thereof, 40 mg of mannitol, 10 mg of sucrose, 0.765 mg of tromethamine, hydrochloric acid, and the balance made up of water; and wherein the pharmaceutical preparation has a pH value of 6.5 to 8.5, preferably 6.9 to 8.0.

17. The pharmaceutical preparation according to any one of claims 1 to 16, **characterized in that** the pharmaceutical preparation is sterile.

18. The pharmaceutical preparation according to any one of claims 1 to 16, **characterized in that** the pharmaceutical preparation is stable during freezing and thawing.

19. A preparation method for preparing the pharmaceutical preparation according to any one of claims 1 to 18, **characterized in that** the preparation method comprises the following steps:
using a prescription amount of an active drug and optionally adding a pharmaceutically acceptable adjuvant, and carrying out uniform mixing to obtain the pharmaceutical preparation.

20. The preparation method according to claim 19, **characterized in that** the preparation method comprises the following steps: using a prescription amount of a ligand-drug conjugate or a pharmaceutically acceptable salt thereof, a lyophilized excipient, a buffering agent and a solvent, dissolving the ligand-drug conjugate or the pharmaceutically acceptable salt thereof and the lyophilized excipient using the solvent, adjusting the pH value to 6.9 to 7.7 using the buffering agent, carrying out uniform mixing, and carrying out filtering to obtain the pharmaceutical preparation.

21. A freeze-dried preparation, **characterized in that** the freeze-dried preparation is prepared by freeze-drying the pharmaceutical preparation according to any one of claims 1 to 18.

22. A preparation method for preparing the freeze-dried preparation according to claim 21, **characterized in that** the preparation method comprises the following steps:
(i) freezing the pharmaceutical preparation at -45°C;
(ii) annealing the pharmaceutical preparation at -25°C;
(iii) re-freezing the pharmaceutical preparation at -45°C;
(iv) carrying out first drying on the pharmaceutical preparation at -15°C;
and (v) carrying out second drying on the pharmaceutical preparation at 25°C.

23. The preparation method according to claim 22, **characterized in that** the time of the first drying is 800 to 3000 min; and the time of the second drying is 300 to 600 min.

24. A liquid preparation, **characterized in that** the liquid preparation is reconstituted by the freeze-dried preparation according to claim 21 using water.

25. The liquid preparation according to claim 24, **characterized in that** the water comprises one or more of distilled water, pure water, and sterile water; and preferably, the freeze-dried preparation has a pH value of 6.5 to 8.5 after being reconstituted using water.

26. A preparation method for preparing the liquid preparation according to claim 24 or 25, **characterized in that** the preparation method comprises the following steps:
mixing the freeze-dried preparation with sterile water to obtain the liquid preparation.

27. A drug-containing delivery device, **characterized in that** the drug-containing delivery device comprises one of the following preparations: the pharmaceutical preparation according to any one of claims 1 to 18, the freeze-dried preparation according to claim 21, or the liquid preparation according to claim 24 or 25.

28. A pre-filled syringe, **characterized in that** the pre-filled syringe comprises one of the following preparations: the pharmaceutical preparation according to any one of claims 1 to 18, the freeze-dried preparation according to claim 21 or the liquid preparation according to claim 24 or 25, preferably for use in intravenous injection or intramuscular injection.

29. Use of the pharmaceutical preparation according to any one of claims 1 to 18, the freeze-dried preparation according to claim 21, or the liquid preparation according to claim 24 or 25 in the preparation of a delivery device or a pre-filled syringe or a drug for enhancing an immune effector cell response and/or reducing immunosuppression in a subject.

30. Use of the pharmaceutical preparation according to any one of claims 1 to 18, the freeze-dried preparation according to claim 21, or the liquid preparation according to claim 24 or 25 in the preparation of a delivery device or a pre-filled syringe or a drug for the treatment or prevention of a cancer, an immune disease, a cardiovascular disease, a metabolic disease and a neurological disease in a subject.

31. The use according to claim 30, **characterized in that**
the cancer comprises one or more of breast cancer, lung cancer, prostate cancer, kidney cancer, leukemia, ovarian cancer, stomach cancer, uterine cancer, endometrial cancer, liver cancer, colon cancer, thyroid cancer, pancreatic cancer, colorectal cancer, esophageal cancer, skin cancer, lymphoma, and multiple myeloma;
the immune disease comprises one or more of a connective tissue disease, systemic sclerosis, rheumatoid arthritis, and systemic lupus erythematosus;
the cardiovascular disease comprises one or more of angina pectoris, myocardial infarction, apoplexy, heart attack, a hypertensive heart disease, a rheumatic heart disease, cardiomyopathy, cardiac arrhythmia, and a congenital heart disease;
the metabolic disease comprises one or more of diabetes mellitus, gout, obesity, hypoglycemia, hyperglycemia, and dyslipidemia;
and the neurological disease comprises one or more of Alzheimer's disease, Parkinson's disease, Huntington's disease, head injury, multiple sclerosis, vertigo, coma, and epilepsy.
